# EUROPEAN PATENT SPECIFICATION

(11) **EP 0 371 492 B1**
(45) Date of publication and mention of the grant of the patent: **21.06.1995**
(21) Application number: 89122039.4
(22) Date of filing: 29.11.1989
(51) Int. Cl.: C07D 211/90

(54) **Process for the preparation of 1,4-dihydropyridine derivatives**
Verfahren zur Herstellung von 1,4-Dihydropyridin-Derivaten
Procédé pour la préparation de dérivés de 1,4-dihydropyridine

(30) Priority: 30.11.1988 JP 301075/88
(43) Date of publication of application: 06.06.1990
(73) Proprietor: NISSHIN FLOUR MILLING CO., LTD., Chuo-ku, Tokyo 103 (JP)
(72) Inventor: Satoh, Hiroaki, Iruma-gun Saitama-ken (JP); Himoto, Shizuo, Kawagoe-shi Saitama-ken (JP)
(74) Representative: Türk, Gille, Hrabal, Leifert

(56) References cited:
- EP-A- 0 088 276
- EP-A- 0 097 821
- EP-A- 0 153 016
- EP-A- 0 244 595
- EP-A- 0 287 828
- GB-A- 2 158 065
- CHEM. PHARM. BULL., vol. 27, no. 6, 1979, pages 1426-1440; M. IWANAMI et al.: "Synthesis of new water-soluble dihydropyridine vasodilators"

## Description

### FIELD OF THE INVENTION

This invention relates to a process for the preparation of 1,4-dihydropyridine derivatives having a 2-haloethoxycarbonyl group at the 3-position which are useful as an intermediate compound for the synthesis of 2,6-dimethyl-4-phenyl-1,4-dihydropyridine-3,5-dicarboxylic acid diesters having valuable vasodilating and blood pressure lowering effects, etc.

### BACKGROUND OF THE INVENTION

Diethyl 2,6-dimethyl-4-phenyl-1,4-dihydropyridine-3,5-dicarboxylate is known to obtain by reacting ethyl 2-benzylidene-3-oxobutanoate, ethyl 3-aminocrotonate or ethyl 3-oxobutanoate and ammonia, as reported in Ber. Deutsch Chem. Ges. 31 743 (1971). DOS 2117517 and 2117573 disclose that similar compounds can be used as coronary arteriodilating and antihypertensive agents, and inter alia dimethyl 2,6-dimethyl-4-(2-nitrophenyl)-1,4-dihydropyridine-3,5-dicarboxylate disclosed therein has been used extensively under the name of Nifedipine. Since commercial success of Nifedipine, a large number of compounds having similar chemical structure have been developed and these compounds are disclosed in US Patents 3,574,843; 4,264,611; 3,799,934 4,239,893; 4,317,768; 4,044,141 and 4,258,042; EPO Appln. 0012180; and French Patent 2,182,98.

We have developed 1,4-dihydropyridine derivatives wherein a heterocyclic group is linked to an ethylene group through an ester bond in an ester moiety at the 3-position of 2,6-dimethyl-4-phenyl-1,4-dihydropyridine-3,5-dicarboxylic acid diesters and processes for the preparation thereof, which are disclosed in Japanese Patent LOP Publication Nos. 174070/1987 and 292781/1987. The 1,4-dihydropyridine derivatives having a 2-haloethoxycarbonyl group at the 3-position as their intermediates are synthesized by a simultaneous reaction of an aromatic aldehyde, a 2-haloethyl 3-oxobutanoate and a 3-aminocrotonic acid ester in an alcohol solvent (Japanese Patent LOP Publication Nos. 9083/1980 and 174070/1987) or by a stepwise reaction which comprises reacting an aromatic aldehyde with a 2-haloethyl 3-oxobutanoate to form a 2-haloethyl 2-benzylidene-3-oxobutanoate and then reacting 2-benzylidene-3-oxobutanoate with a 3-aminocrotonic acid ester (Japanese Patent LOP Publication Nos. 54318/1985 and 199874/1985).

However, the former method of reacting in one step three materials of an aromatic aldehyde, a 2-haloethyl 3-oxobutanoate and a 3-aminocrotonic acid ester forms a large amount of by-products and is not practical. The latter method by the stepwise reaction is of the drawback that a water molecule forming during the reaction hydrolyses a 2-haloethyl group to a 2-hydroxyethyl group which results in reducing a yield of the aimed compound having a 2-haloethoxycarbonyl group at the 3-position of the 1,4-dihydropyridine ring. Thus a process for the production of these intermediate compounds in higher yield has been demanded.

We have extensively studied the above-mentioned latter process by the stepwise reaction in an effort to overcome the drawbacks and found that the aimed 1,4-dihydropyridine derivatives containing a 2-haloethoxycarbonyl group are produced in high yield by carrying out the reaction of a 2-haloethyl 3-oxobutanoate and a 3-aminocrotonic acid ester in the presence of a dehydrating agent.

Thus the present invention provides a process for the preparation of 1,4-dihydropyridine derivatives of formula I 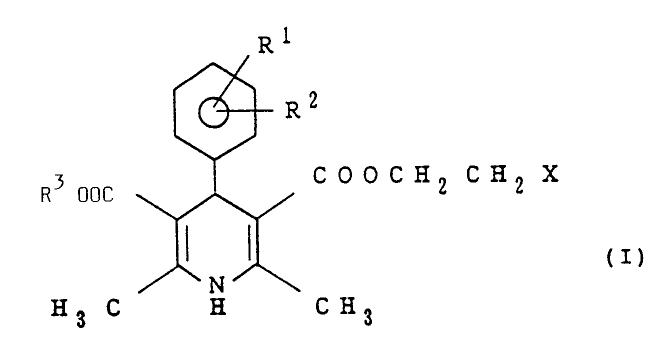
wherein R¹ and R² may be the same or different and each is hydrogen, halogen, trifluoromethyl or nitro but both are not hydrogen, X is halogen and R³ is C₁-C₆ alkyl or C₁-C₆ alkoxyalkyl which comprises reacting an aromatic aldehyde of formula II 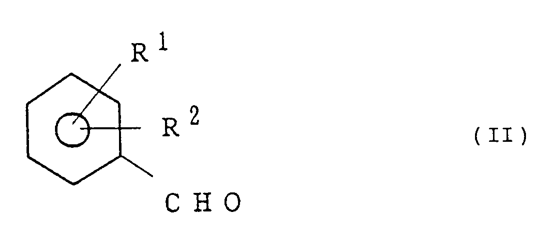
wherein R¹ and R² have the same meaning as defined above with a 2-haloethyl 3-oxobutanoate of formula III 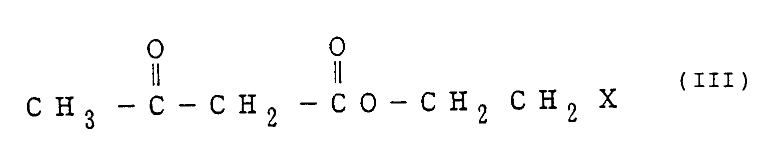
wherein X has the same meaning as defined above to form a 2-haloethyl 2-benzylidene-3-oxobutanoate of formula IV 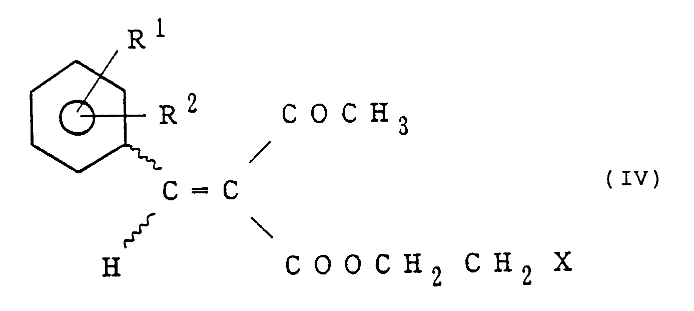
wherein R¹, R² and X have the same meaning as defined above and then reacting the 2-haloethyl 2-benzylidene-3-oxobutanoate with a 3-aminocrotonic acid ester of formula V 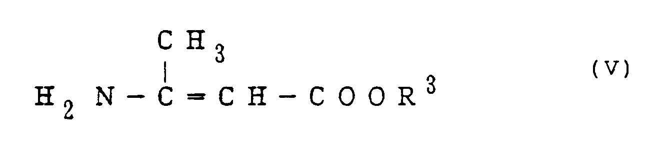
in a reaction medium in the presence of a dehydrating agent.

The aromatic aldehydes of formula II which are used in the process of the invention include 3-nitrobenzaldehyde, 2-nitrobenzaldehyde, 2,3-dichlorobenzaldehyde, 2-trifluoromethylbenzaldehyde, 4-nitrobenzaldehyde, 3-trifluoromethylbenzaldehyde, 4-trifluoromethylbenzaldehyde or the like. The 2-haloethyl 3-oxobutanoate of formula III include 2-chloroethyl 3-oxobutanoate, 2-bromoethyl 3-oxobutanoate, 2-iodoethyl 3-oxobutanoate, 2-fluoroethyl 3-oxobutanoate or the like. The 3-aminocrotonic acid esters of formula V include methyl 3-aminocrotonate, ethyl 3-aminocrotonate, isopropyl 3-aminocrotonate, n-propyl 3-aminocrotonate, isobutyl 3-aminocrotonate, n-butyl 3-aminocrotonate, sec-butyl 3-aminocrotonate, tert-butyl 3-aminocrotonate, 2-methoxyethyl 3-aminocrotonate, methoxypropyl 3-aminocrotonate, ethoxyethyl 3-aminocrotonate, or the like.

In the process of the invention, the compound of formula II and the compound of formula III are used in a molar ratio of 1.0 : 0.1-10, especially preferably 1.0 : 0.8-1.2. The compound of formula IV and the compound of formula V are used in a molar ratio of 1.0 : 0.1-10, especially preferably 1.0 : 0.8-1.2.

The reaction between the compound of formula II and the compound of formula III is usually carried out at a reaction temperature of 0° to 150°C and a reaction time of one hour to three days without any solvent or in a reaction medium such as an aromatic hydrocarbon solvent e.g. benzene, toluene, xylene, an aliphatic hydrocarbon solvent e.g. hexane, cyclohexane, ligroin, an ether solvent e.g. isopropyl ether, diethyl ether, tetrahydrofuran, dioxane, methyl cellosolve, a halogenated hydrocarbon solvent e.g. methylene chloride, chloroform, trichlene, an alcohol solvent e.g. methanol, ethanol, isopropyl alcohol in the presence of an acid catalyst such as hydrogen chloride, sulfuric acid, acetic acid or a basic catalyst such as diisopropylamine, pyridine, piperidine, triethylamine or a mixture of these acid catalysts or a mixture of these basic catalysts.

A condensation reaction between the compound of formula IV and the compound of formula V is usually performed at a reaction temperature of 0°C to 150°C and a reaction time of one hour to three days in a reaction medium such as methanol, ethanol, isopropyl alcohol, n-butyl alcohol, toluene, benzene, cyclohexane alone or in combination.

In the feature of the invention carrying out a condensation reaction between the compound of formula IV and the compound of formula V in the presence of a dehydrating agent, the dehydrating agents used in the reaction include anhydrous sodium sulfate, anhydrous magnesium sulfate, molecular sieve 4A, molecular sieve 3A or the like, molecular sieve 3A being especially preferred.

The dehydrating agent is used in a proportion of 50-500 grams per mole of the compound of formula IV.

The compound of formula IV prepared by reaction of the compound of formula II with the compound of formula III may be isolated and condensed with the compound of formula V, but may be subjected to a reaction with the compound of formula V without isolation.

The compound of formula IV can be isolated by extraction, column chromatography, crystallization or the like.

The compound of formula I prepared by a condensation reaction between the compound of formula IV and the compound of formula V can be isolated by extraction, column chromatography, crystallization or the like.

According to the present process wherein 2-haloethyl 2-benzylidene-3-oxobutanoate prepared by reaction of aromatic aldehydes with 2-haloethyl 3-oxobutanoate are reacted with 3-aminocrotonic acid esters in the presence of the dehydrating agent, there can be prepared in good yield the desired 1,4-dihydropyridine derivatives having 2-haloethoxycarbonyl group at the 3-position.

The invention is further illustrated by the following examples.

### EXAMPLE 1

### 2-Chloroethyl methyl 2,6-dimethyl-4-(3-nitropbenyl)-1,4-dihydropyridine-3,5-dicarboxylate

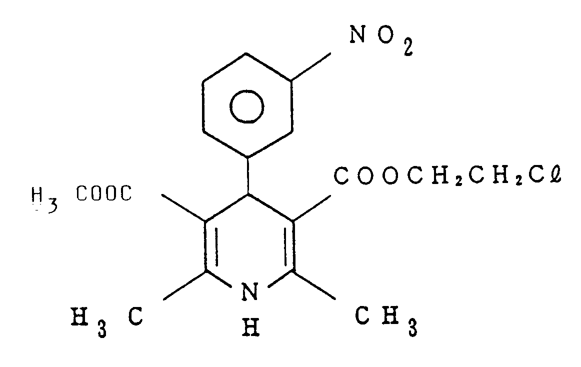

2-Chloroethyl 2-(3-nitrobensylidene)-3-oxobutanoate (8.00 g) and methyl 3-aminocrotonate (3.40 g) were charged into a 100 ml three necked flask and dissolved in 30 ml of isopropyl alcohol. A Soxhlet extractor containing 5 g of molecular sieve 3A and a reflux condenser were attached to the flask and a reaction mixture was heated under reflux for 3 hrs. After completion of the reaction, 2-propanol was distilled off, the reaction mixture was dissolved in chloroform and washed with diluted hydrochloric acid and water. It was dried over magnesium sulfate, separated and purified by column chromatography and crystallized from ethyl acetate/hexane. The crystals were filtered and dried under reduced pressure to afford 10.16 g (96% yield) of the title compound, m.p. 134-137°C.

For comparison, the reaction was carried out without using molecular sieve in the above manner. The yield was 84% with the formation of a hydrolyzed compound, 2-hydroxyethyl methyl 2,6-dimethyl-4-(3-nitrophenyl)-1,4-dihydropyridine-3,5-dicarboxylate (10% yield, m.p. 180-182°C) as by-product.

### EXAMPLE 2

### 2-Chloroethyl methyl 2,6-dimethyl-4-(2-nitrophenyl)-1,4-dihydropyridine-3,5-dicarboxylate

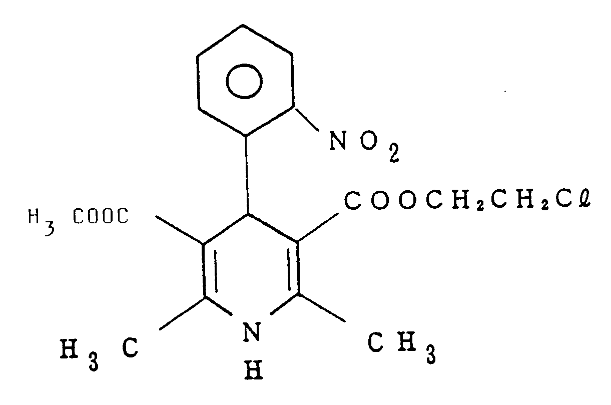

2-Chloroethyl 2-(2-nitrobenzylidene)-3-oxobutanoate (8.00 g) and methyl 3-aminocrotonate (3.40 g) were reacted in a similar manner as in Example 1 to afford 8.44 g (80% yield) of the title compound, m.p. 117-122°C.

For comparison, the reaction was carried out without using dehydrating agent in the above manner. The yield was 59% with the formation of a hydrolyzed compound, 2-hydroxyethyl methyl 2,6-dimethyl-4-(2-nitrophenyl)-1,4-dihydropyridine-3,5-dicarboxylate (11% yield, m.p. 127-129°C) as by-product.

### EXAMPLE 3

### 2-Chloroethyl methyl 2,6-dimethyl-4-(2-trifluoromethylphenyl)-1,4-dihydropyridine-3,5-dicarboxylate

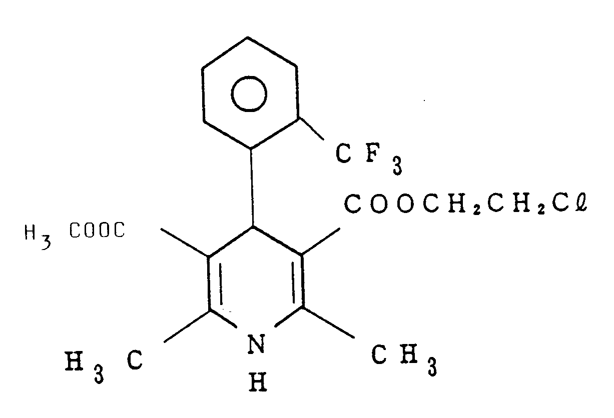

2-Chloroethyl 3-oxo-2-(2-trifluoromethylbenzylidene)butanoate (19.24 g) and methyl 3-aminocrotonate (7.60 g) were reacted in the presence of molecular sieve 3A (10 g) in a similar manner as in Example 1 to give 20.38 g (81% yield) of the title compound, m.p. 132-134°C.

For comparison, the reaction was carried out without using dehydrating agent in the above manner. The yield was 57% with the formation of a hydrolyzed compound, 2-hydroxyethyl methyl 2,6-dimethyl-4-(2-trifluoromethylphenyl)-1,4-dihydropyridine-3,5-dicarboxylate (16% yield, m.p. 141-144°C) as by-product.

2-Chloroethyl 3-oxo-2-(2-trifluoromethylbenzylidene)butanoate used in this example was prepared in the following manner.

To a cyclohexane (1 lit.) solution of 2-trifluoromethyl benzaldehyde (250 g) and 2-chloroethyl 3-oxobutanoate (260 g) was added sulfuric acid (4 ml), a reaction mixture was heated under reflux for 5 hrs. and an azeotropically producing water was removed with a water separator. After completion of the reaction, it was treated in a conventional way to afford 373 g of 2-chloroethyl 3-oxo-2-(2-trifluoromethylbenzylidene)butanoate.

### EXAMPLE 4

### 2-Chloroethyl methyl 2,6-dimethyl-4-(2,3-dichlorophenyl)-1,4-dihydropyridine-3,5-dicarboxylate

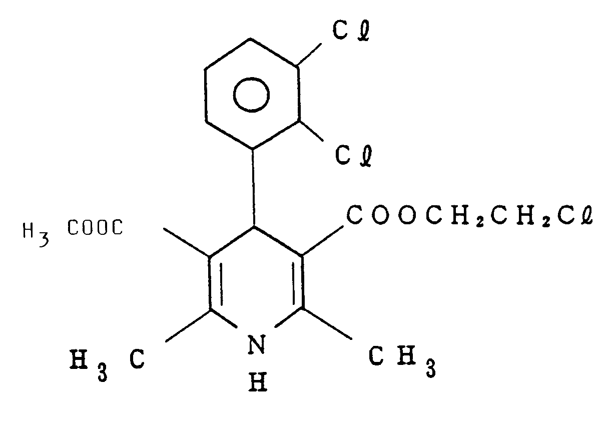

2-Chloroethyl 2-(2,3-dichlorobenzylidene)-3-oxobutanoate (8.64 g) and methyl 3-aminocrotonate (3.40 g) were reacted in a similar manner as in Example 1 to give 8.91 g (79% yield) of the title compound, m.p. 141-145°C.

For comparison, the reaction was carried out without using dehydrating agent in the above manner. The yield was 64% with the formation of a hydrolyzed compound, 2-hydroxyethyl methyl 2,6-dimethyl-4-(2,3-dichioropbenyl)-1,4-dihydropyridine-3,5-dicarboxylate (11% yield, m.p. 113-118°C) as by-product.

### EXAMPLE 5

### 2-Chloroethyl methyl 2,6-dimethyl-4-(2-trifluoromethylphenyl)-1,4-dihydropyridine-3,5-dicarboxylate

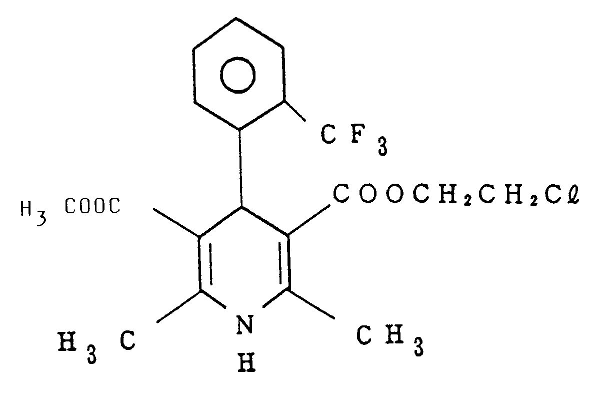

2-Chloroethyl 3-oxo-2-(2-trifluoromethylbenzylidene)butanoate (19.24 g) and methyl 3-aminocrotonate (3.40 g) were reacted in the presence of anhydrous magnesium sulfate (7.3 g) in a similar manner as in Example 1 to give 16.62 g (66% yield) of the title compound, m.p. 132-134°C.

## Claims

1. A process for the preparation of 1,4-dihydropyridine derivatives of formula I 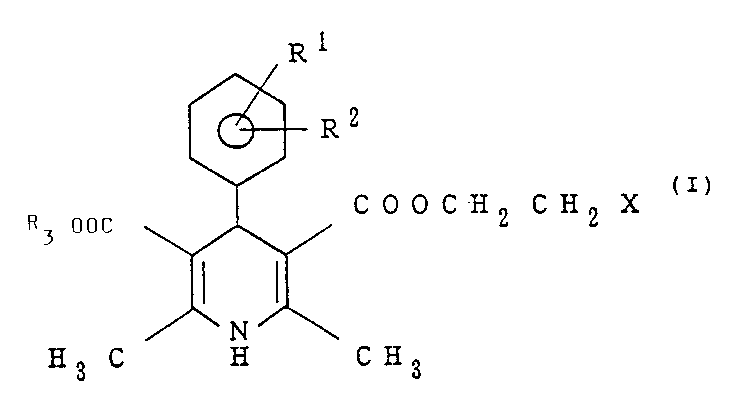 wherein R¹ and R² may be the same or different and each is hydrogen, halogen, trifluoromethyl or nitro but both are not hydrogen, X is halogen and R³ is C₁-C₆ alkyl or C₁-C₆ alkoxyalkyl characterized by reacting an aromatic aldehyde of formula II 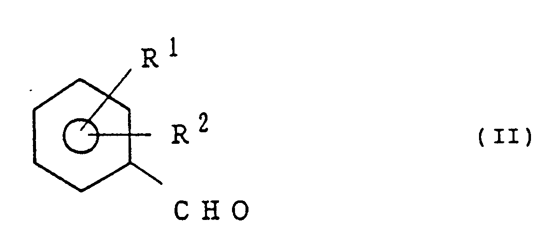 wherein R¹ and R² have the same meaning as defined above with a 2-haloethyl 3-oxobutanoate of formula III 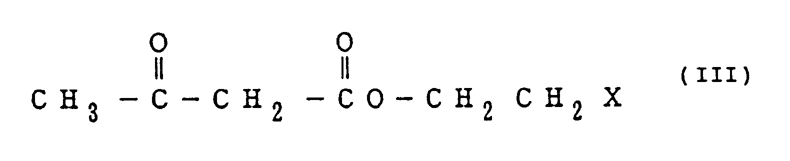 wherein X has the same meaning as defined above to form a 2-haloethyl 2-benzylidene-3-oxobutanoate of formula IV 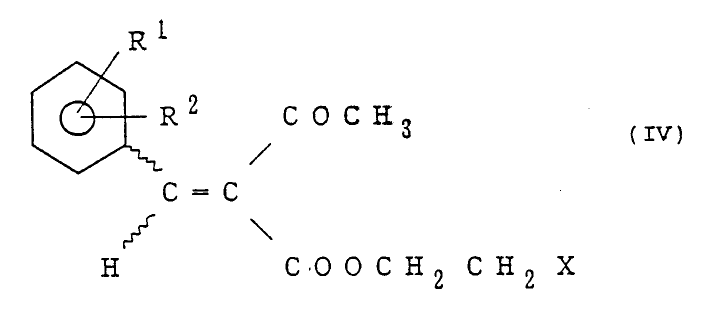 wherein R¹, R² and X have the same meaning as defined above and then reacting the 2-haloethyl 2-benzylidene-3-oxobutanoate with a 3-aminocrotonic acid ester of formula V 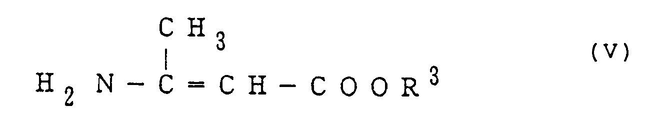 in a reaction medium in the presence of a dehydrating agent.

2. A process of Claim 1 wherein a molar ratio of the compound of formula II to that of formula III is 1.0 : 0.1-10, especially preferably 1.0 : 0.8-1.2.

3. A process of Claim 1 or 2 wherein a molar ratio of the compound of formula IV to that of formula V is 1.0 : 0.1-10, especially preferably 1.0 : 0.8-1.2.

4. A process of Claim 1, 2 or 3 wherein a condensation reaction between the compound of formula IV and that of formula V is usually performed at a reaction temperature of 0°C to 150°C and a reaction time of one hour to three days.

5. A process of Claim 1, 2, 3 or 4 wherein the dehydrating agent includes anhydrous sodium sulfate, anhydrous magnesium sulfate, molecular sieve 4A or molecular sieve 3A.

6. A process of Claim 1, 2, 3, 4 or 5 wherein the dehydrating agent is used in a proportion of 50-500 grams per mole of the compound of formula IV.

## Patentansprüche

1. Verfahren zur Herstellung von 1,4-Dihydropyridinderivaten der Formel (I) 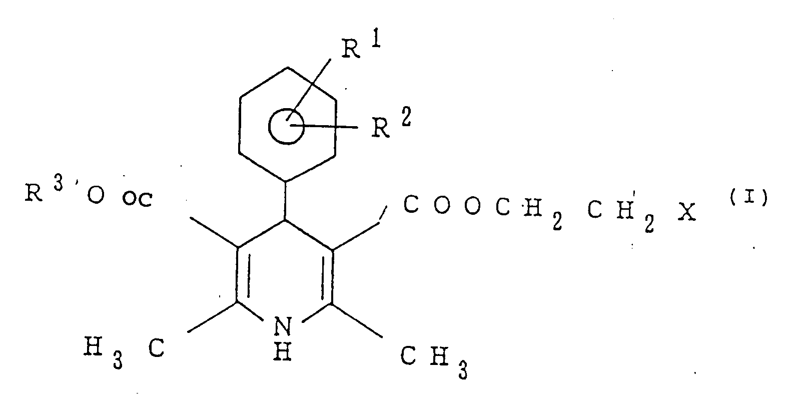 worin bedeuten:
R¹ und R², die gleich oder verschieden sein können, jeweils Wasserstoff, Halogen, Trifluoromethyl oder Nitro, wobei jedoch nicht beide Wasserstoff sein können,
X Halogen und
R³ C₁-C₆-Alkyl oder C₁-C₆-Alkoxyalkyl,
dadurch gekennzeichnet, daß man einen aromatischen Aldehyd der Formel (II) 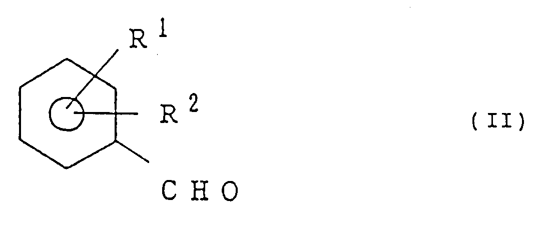 worin R¹ und R² die gleichen Bedeutungen wie oben angegeben haben, mit einem 2-Halogenethyl-3-oxobutanoat der Formel (III) umsetzt 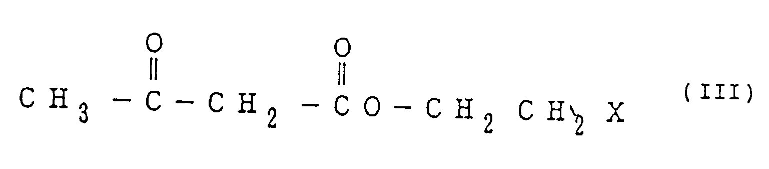 worin X die gleiche Bedeutung wie oben angegeben hat,
unter Bildung eines 2-Halogenethyl-2-benzyliden-3-oxobutanoats der Formel (IV) 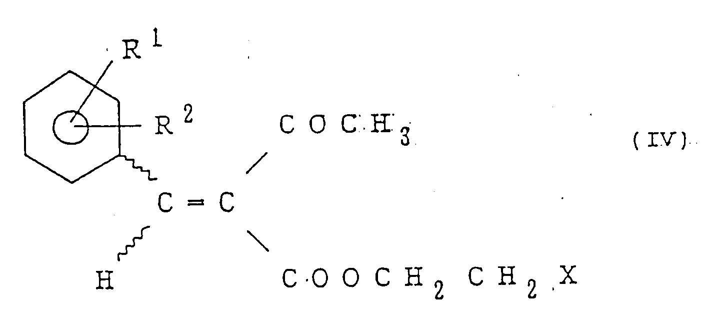 worin R¹, R² und X die gleiche Bedeutung wie oben angegeben haben, und
anschließende Umsetzung des 2-Halogenethyl-2-benzyliden-3-oxobutanoats mit einem 3-Aminocrotonsäureester der Formel (V) 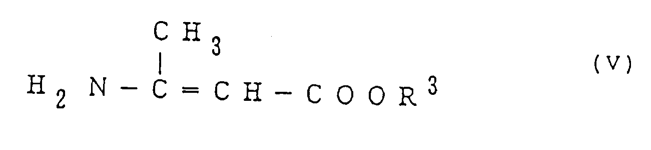 in einem Reaktionsmedium in Gegenwart eines Dehydratisierungsmittels.

2. Verfahren nach Anspruch 1, worin das Molverhältnis zwischen der Verbindung der Formel (II) und der Verbindung der Formel (III) 1,0:0,1 bis 10, besonders bevorzugt 1,0:0,8 bis 1,2, beträgt.

3. Verfahren nach Anspruch 1 oder 2, worin das Molverhältnis zwischen der Verbindung der Formel (IV) und der Verbindung der Formel (V) 1,0:0,1 bis 10, besonders bevorzugt 1,0:0,8 bis 1,2, beträgt.

4. Verfahren nach Anspruch 1, 2 oder 3, worin die Kondensationsreaktion zwischen der Verbindung der Formel (IV) und der Verbindung der Formel (V) in der Regel bei einer Reaktionstemperatur von 0 bis 150°C und einer Reaktionszeit von 1 h bis 3 Tagen durchgeführt wird.

5. Verfahren nach Anspruch 1, 2, 3 oder 4, worin das Dehydratisierungsmittel beispielsweise umfaßt wasserfreies Natriumsulfat, wasserfreies Magnesiumsulfat, das Molekularsieb 4A oder das Molekularsieb 3A.

6. Verfahren nach Anspruch 1, 2, 3, 4 oder 5, worin das Dehydratisierungsmittel in einem Mengenanteil von 50 bis 500 g pro mol der Verbindung der Formel (IV) verwendet wird.

## Revendications

1. Procédé pour la préparation de dérivés de dihydro-1,4 pyridine de la formule I 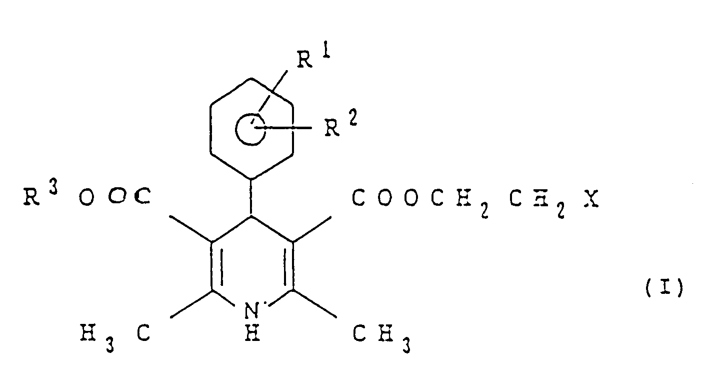 où R¹ et R² peuvent être identiques ou différents et chacun est un hydrogène, un halogène, du trifluorométhyle ou un groupement nitro, mais les deux ne sont pas ensemble, un hydrogène, X est un halogène et R³ est un alkyle C₁-C₆ ou un alcoxyalcoyle C₁-C₆, caractérisé par la réaction d'un aldéhyde aromatique de formule II 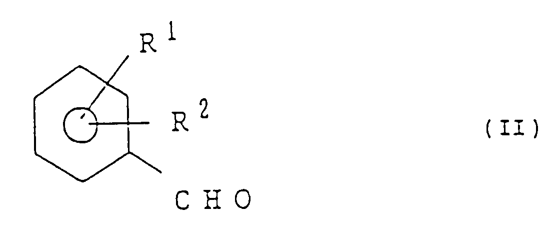 où R¹ et R² ont la même signification que celle définie précédemment avec un oxo-3 butanoate d'halo-2 éthyle de formule III 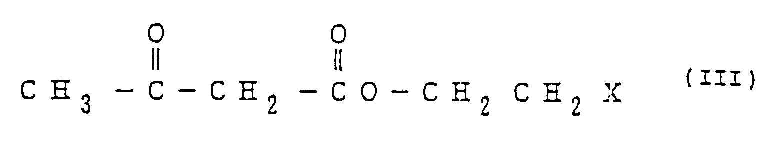 où X a la même signification que celle définie précédemment pour former un oxo-3 butanoate d'halo-2 éthylbenzylidène-2 de formule IV 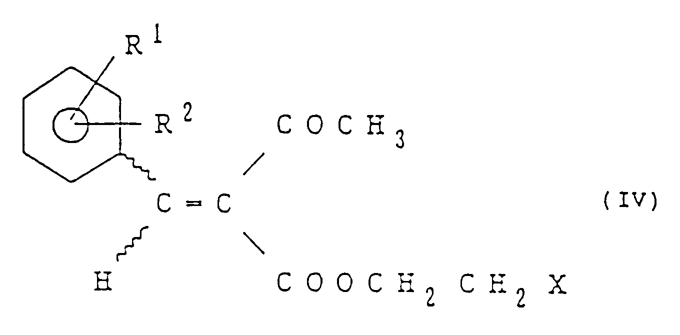 où R¹, R² et X ont la même signification que celle définie précédemment et l'oxo-3 butanoate d'halo-2 éthylbenzylidène-2 réagissant alors avec un ester d'acide amino-3 crotonique de formule V 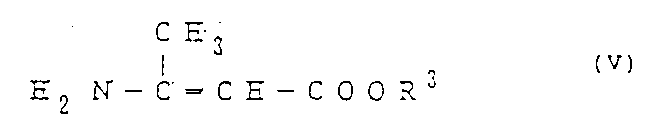 dans un milieu réactif en présence d'un agent déshydratant.

2. Procédé selon la revendication 1, où un rapport moléculaire du composé de formule II par rapport à celui de formule III est de 1,0 : 0,1-10, de préférence de 1,0 : 0,8-1,2.

3. Procédé selon la revendication 1 ou 2, où un rapport moléculaire du composé de formule IV par rapport à celui de formule V est de 1,0 : 0,1-10, de préférence de 1,0 : 0,8-1,2.

4. Procédé selon la revendication 1, 2 ou 3, où une réaction de condensation entre le composé de formule IV et celui de formule V est effectuée habituellement à une température de réaction de 0°C à 150°C et suivant une durée de réaction comprise entre 1 heure et 3 jours.

5. Procédé selon la revendication 1, 2, 3 ou 4, où l'agent déshydratant comprend du sulfate de sodium anhydre, du sulfate de magnésium anhydre, un tamis moléculaire 4A ou un tamis moléculaire 3A.

6. Procédé selon la revendication 1,2,3,4 ou 5, où l'agent déshydratant est utilisé suivant une proportion de 50 à 500 grammes par mole du composé de formule IV.
